# EUROPEAN PATENT SPECIFICATION

(11) **EP 2 905 341 B1**
(45) Date of publication and mention of the grant of the patent: **29.04.2020**
(21) Application number: 14154335.5
(22) Date of filing: 07.02.2014
(51) Int. Cl.: C12P 19/30, C12P 19/18

(54) **Methods for producing GDP-fucose using nucleotide sugar transporters and recombinant microorganism host cells used therefor**
Verfahren zur Herstellung von GDP-Fucose mit Hilfe von Nukleotid-Zuckertransporter und rekombinante Mikroorganismus-Wirtszellen dafür
Procédés de production de GDP-fucose à l'aide des transporteurs de sucre nucléotidique et cellules hôtes de micro-organisme recombinant utilisées à cet effet

(43) Date of publication of application: 12.08.2015
(73) Proprietor: Jennewein Biotechnologie GmbH, 53619 Rheinbreitbach (DE)
(72) Inventor: Dr. Albermann, Christoph, 70569 Stuttgart (DE); Prof. Dr. Sprenger, Georg, 71522 Backnang (DE); Dr. Jennewein, Stefan, 53604 Bad Honnef (DE)
(74) Representative: Witte, Weller & Partner Patentanwälte mbB

(56) References cited:
- WO-A1-2010/036898
- WO-A1-2010/142305
- US-A- 5 334 514
- US-A- 5 824 472
- US-A1- 2006 099 680
- MAGGIONI ET AL: "Targeting the expression of functional murine CMP-sialic acid transporter to the E. coli inner membrane", BIOCHEMICAL AND BIOPHYSICAL RESEARCH COMMUNICATIONS, ACADEMIC PRESS INC. ORLANDO, FL, US, vol. 362, no. 3, 11 September 2007 (2007-09-11), pages 779-784, XP022241439, ISSN: 0006-291X, DOI: 10.1016/J.BBRC.2007.08.070
- Shigemi Wada ET AL: "Functional Expression of the Human UDP-Galactose Transporters in the Yeast Saccharomyces cerevisiae 1 Ge-Hong Sun", J. Biochem, 1 January 1998 (1998-01-01), pages 912-917, XP055123881, Retrieved from the Internet: URL:http://jb.oxfordjournals.org/content/1 23/5/912.full.pdf [retrieved on 2014-06-17]
- HIROAKI SEGAWA ET AL: "Human and Drosophila UDP-galactose transporters transport UDP-N-acetylgalactosamine in addition to UDP-galactose", EUROPEAN JOURNAL OF BIOCHEMISTRY, vol. 269, no. 1, 1 January 2002 (2002-01-01), pages 128-138, XP055123893, ISSN: 0014-2956, DOI: 10.1046/j.0014-2956.2001.02632.x
- BAKKER H ET AL: "A CMP-sialic acid transporter cloned from Arabidopsis thaliana", CARBOHYDRATE RESEARCH, PERGAMON, GB, vol. 343, no. 12, 11 August 2008 (2008-08-11), pages 2148-2152, XP022757852, ISSN: 0008-6215, DOI: 10.1016/J.CARRES.2008.01.010 [retrieved on 2008-01-17]
- A. ASHIKOV ET AL: "The Human Solute Carrier Gene SLC35B4 Encodes a Bifunctional Nucleotide Sugar Transporter with Specificity for UDP-Xylose and UDP-N-Acetylglucosamine", JOURNAL OF BIOLOGICAL CHEMISTRY, vol. 280, no. 29, 23 May 2005 (2005-05-23) , pages 27230-27235, XP055123894, ISSN: 0021-9258, DOI: 10.1074/jbc.M504783200
- KAZUHIKO TABATA ET AL: "Production of UDP-N-acetylglucosamine by coupling metabolically engineered bacteria", BIOTECHNOLOGY LETTERS, KLUWER ACADEMIC PUBLISHERS, DORDRECHT, vol. 22, no. 6, 1 March 2000 (2000-03-01), pages 479-483, XP008145965, ISSN: 0141-5492, DOI: 10.1023/A:1005627820455 [retrieved on 2004-12-22]

## Description

The present invention relates to methods for producing GDP-fucose in a recombinant prokaryotic microorganism host cell, as well as to vectors and recombinant prokaryotic microorganism host cells suitable for the production of GDP-fucose.

Sugars - also referred to as oligo- and mono-saccharides - fulfill a multitude of vital functions in the human body. The most commonly known function of sugar is to supply the organism with the required energy. Beside the function of energy supply, which is generally accomplished by widely known molecules such as fructose and saccharose, it is known today that specific sugar-structures accomplish further vital function in the human body. For example, it has been shown that the health-protecting function of human mother milk can be attributed to specific complex sugar molecules.

Within the saccharides, nucleotide-activated sugars represent a class of glycoconjugates that are high energy cosubstrates involved in carbohydrate biosynthetic reactions. They are glycosyl-group donors for the glycosyltransferases that synthesize - depending on the producing cell - N- and O-linked oligosaccharide chains of glycoproteins, proteoglycans, glycosaminoglycans, cell wall polysaccharides, lipopolysaccharides, capsular polysaccharides, and that catalyze the glycosylation of metabolites in the cell.

Nucleotide-activated monosaccharides, or modified forms thereof, (particularly sugars, such as GDP-fucose, GDP-glucose, GDP-mannose, GDP-arabinose, ADP-glucose, UDP-glucose, UDP-N-acetylglucosamine, UDP-galactose, UDP-N-acetylgalactosamine, UDP-glucuronic acid, UDP-xylose, CMP-sialic acid, etc.) play an important role during enzymatic biosynthesis of glycosylated oligosaccharides and glycoconjugates.

E.g., enzymatic syntheses of complex fucose-containing glycans require the nucleotide-activated molecule GDP-fucose. A great variety of functional fucosylated glycans can be produced by the utilization of GDP-fucose. Fields of application for fucosylated compounds are, for example the synthesis of fucosylated oligosaccharides, such as fucosylated human milk oligosaccharides, the development of cancer vaccines, the development of biomimetics for the suppression of diseases like asthma and chronic obstructive pulmonary disease, the development of glycoconjugate drugs with increased bioactivity and stability, etc.

The carbohydrate moiety provides many biological functions. Natural carbohydrates have complex structures including many kinds of monosaccharide units, which are very difficult and costly to generate by chemical synthetic approaches. Chemical syntheses represent very time and cost intensive methods, and appear - in particular in view of a technical application - to be very inefficient, e.g., by using solid-phase synthesis. In recent approaches, as an alternative, nucleotide-activated sugars have been generated by enzymatic *in vitro* syntheses, in de novo pathways or salvage pathways in recombinant bacteria.

However, up to now, the production of nucleotide-activated sugars, monosaccharides, in particular of hexoses, or modified forms thereof, in large scale has only been accomplished with high and intensive efforts in view of the material and time needed, and an efficient, time- and cost-saving production method for nucleotide-activated monosaccharides is highly desirable. E.g., Maggioni et al. ("Targeting the expression of functional murine CMP-sialic acid transporter to the E. coli inner membrane" Biochem Bioph Res Co 362 (3):779-784) disclose the expression of the CMP-sialic acid transporter in the *E.coli* inner membrane using the OmpA-signal sequence. The functionality of the recombinant protein inserted within the inner membrane was confirmed by showing the accumulation of CMP-sialic acid *in vitro* in spheroblasted *E coli.* However, Maggioni *et al.* do not disclose a transporter for transporting a nucleotide-activated sugar outside the cytosol of a cell.

Thus, an object of the present invention is the provision of a method for producing GDP-fucose that enables an economically effective large scale production of said GDP-fucose.

According to the invention, this and other objects are solved by a method for producing GDP-fucose in a recombinant prokaryotic microorganism host cell, the method comprising the steps of a) providing a recombinant prokaryotic microorganism host cell, said recombinant prokaryotic microorganism host cell being transformed to comprise at least one nucleic acid sequence heterologous to said recombinant prokaryotic microorganism cell, said nucleic acid heterologous to said recombinant prokaryotic microorganism cell comprising a nucleic acid sequence encoding a transporter protein for GDP-fucose that is fused to a signal sequence which transports the protein through a cellular membrane of the host cell; and b) growing, under suitable nutrient conditions permissive for the production of GDP-fucose, said recombinant prokaryotic host cell under conditions so as to allow said recombinant prokaryotic microorganism host cell to express said transporter protein for GDP-fucose, whereby the GDP-fucose is transported over the membrane; and thereby providing the GDP-fucose in the periplasm of said recombinant prokaryotic microorganism cell or the medium of its growth.

The object is further solved by a genetically modified or recombinant prokaryotic microorganism host cell, the microorganism host cell being transformed to comprise at least one nucleic acid sequence heterologous to the recombinant prokaryotic microorganism host cell, wherein said nucleic acid heterologous to said recombinant prokaryotic microorganism host cell comprises a nucleic acid sequence encoding an eukaryotic transporter protein for a nucleotide-activated sugar that is fused to a signal sequence which transports said protein through a cellular membrane of the host cell, the eukaryotic transporter protein being able to transport the nucleotide-activated sugar from the cytosol across a membrane of said recombinant prokaryotic microorganism host cell, wherein the nucleotide-activated sugar is GDP-fucose.

The invention also relates to the use of such a recombinant prokaryotic microorganism host cell for the production of GDP-fucose.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described. For purposes of the present invention, the following terms are defined below:

A "nucleotide-activated" sugar, i.e. a nucleotide-activated sugar, is presently mean to encompass sugars, or derivatives thereof, that are linked with a nucleotide, i.e. with a nucleoside diphosphate (NDP) or a nucleoside monophosphate (NMP). Also, the expression "sugar" shall presently mean to comprise any monosaccharide having five, six or nine carbon atoms, and encompasses also monosaccharides which carry substitutes in place of one of their hydroxyl groups, e.g. an amine group. A "nucleotide", as generally understood, is composed of a nucleobase, a pentose (ribose or deoxyribose) and at least one phosphate group; as a consequence, a nucleoside triphosphate comprises three phosphate groups, a nucleoside diphosphate comprises two phosphate groups, and a nucleoside monophosphate comprises one phosphate group. The primary nucleo-bases are cytosine, guanine, adenine, thymine and uracil.

As generally understood in the relevant field, a monosaccharide represents the simplest form of carbohydrates and may be subcategorized as a aldose or ketose, and may be further classified based on the number of carbon atoms in the backbone, i.e. tri-(3), tetr-(4), pent-(5), hex-(6), hept-(7), etc. in the name of the sugar.

Accordingly, and also, a "derivative of a sugar" or "derivate of a monosaccharide" is presently, meant to designate sugars or monosaccharides, which carry substitutes in place of one (or more) of their hydroxyl groups. Such sugars or monosaccharides may be, but are not limited, to, sugar/monosaccharide phosphates, deoxy and amino sugars/monosaccharides, sugar alcohols and acids.

In this connection, a nucleotide-activated sugar is GDP-fucose.

In particular, the present invention relates to a method and a use and a genetically modified or recombinant microorganism as mentioned above for producing GDP-fucose.

Presently, the term "nucleic acid" refers to a deoxyribonucleotide or ribonucleotide polymer in either single- or double-stranded form, and unless otherwise limited, encompasses known analogues of natural nucleotides that hybridize to nucleic acids in manner similar to naturally occurring nucleotides. Unless otherwise indicated, a particular nucleic acid sequence includes the complementary sequence thereof.

Presently, the term "operably linked" as used herein, shall mean a functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence. Accordingly, the term "Promoter" designates DNA sequences which usually "precede" a gene in a DNA polymer and provide a site for initiation of the transcription into mRNA. "Regulator" DNA sequences, also usually "upstream" of (i.e., preceding) a gene in a given DNA polymer, bind proteins that determine the frequency (or rate) of transcriptional initiation. Collectively referred to as "promoter/regulator" or "control" DNA sequence, these sequences which precede a selected gene (or series of genes) in a functional DNA polymer cooperate to determine whether the transcription (and eventual expression) of a gene will occur. DNA sequences which "follow" a gene in a DNA polymer and provide a signal for termination of the transcription into mRNA are referred to as transcription "terminator" sequences.

The term "recombinant", as used herein with reference to a microorganism host cell indicates that the microorganism cell replicates a heterologous nucleic acid, or expresses a peptide or protein encoded by a heterologous nucleic acid (i.e., a sequence "foreign to said cell"). Recombinant cells can contain genes that are not found within the native (non-recombinant) form of the cell. Recombinant cells can also contain genes found in the native form of the cell wherein the genes are modified and reintroduced into the cell by artificial means. The term also encompasses cells that contain a nucleic acid endogenous to the cell that has been modified without removing the nucleic acid from the cell; such modifications include those obtained by gene replacement, site-specific mutation, and related techniques. Accordingly, a "recombinant polypeptide" is one which has been produced by a recombinant cell. A "heterologous sequence" or a "heterologous nucleic acid", as used herein, is one that originates from a source foreign to the particular host cell (e.g. from a different species), or, if from the same source, is modified from its original form. Thus, a heterologous nucleic acid operably linked to a promoter is from a source different from that from which the promoter was derived, or, if from the same source, is modified from its original form.

Accordingly, a recombinant prokaryotic microorganism "host cell" is presently understood as a prokaryotic microorganism cell which has been transformed or transfected, or is capable of transformation or transfection by an exogenous polynucleotide sequence.

The art is rich in patent and literature publications relating to "recombinant DNA" methodologies for the isolation, synthesis, purification and amplification of genetic materials for use in the transformation of selected host organisms. Thus, it is common knowledge to transform host organisms with "hybrid" viral or circular plasmid DNA which includes selected exogenous (i.e. foreign or "heterologous") DNA sequences. The procedures known in the art first involve generation of a transformation vector by enzymatically cleaving circular viral or plasmid DNA to form linear DNA strands. Selected foreign DNA strands usually including sequences coding for desired protein product are prepared in linear form through use of the same/similar enzymes. The linear viral or plasmid DNA is incubated with the foreign DNA in the presence of ligating enzymes capable of effecting a restoration process and "hybrid" vectors are formed which include the selected exogenous DNA segment "spliced" into the viral or circular DNA plasmid.

The term "nucleic acid sequence encoding..." generally refers to any polyribonucleotide or polydeoxyribonucleotide, which may be unmodified RNA or DNA or modified RNA or DNA, and generally represents a gene which encodes a certain polypeptide or protein. The term includes, without limitation, single- and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded regions, or a mixture of single- and double-stranded regions. The term also encompasses polynucleotides that include a single continuous region or discontinuous regions encoding the polypeptide (for example, interrupted by integrated phage or an insertion sequence or editing) together with additional regions that also may contain coding and/or non-coding sequences.

As used herein, the term "isolating" means harvesting, recovering, collecting or separating the GDP-fucose produced by the gene expression system from the microorganism recombinant host cell.

With the method according to the invention, an inexpensive and effective means for the production of GDP-fucose is provided, which can be used for producing the desired GDP-fucose in large scale. Laborious and cost-intensive expenditure can, thus, be avoided. The GDP-fucose produced with the method according to the invention can be easily retrieved from the cell or the medium the cell is cultivated in, due to the fact that the host cell employed is expressing a GDP-fucose transporter, which transports the GDP-fucose from the cytosol over a membrane.

Further, the GDP-fucose provided according to the method of the invention in said recombinant prokaryotic microorganism host cell, or in the medium of its growth, can be further used or processed in said recombinant prokaryotic microorganism: The recombinant prokaryotic microorganism host cell can be further genetically modified to further process or use the provided GDP-fucose for producing, e.g., glyocosylated oligosaccharides, in particular fucosylated oligosaccharides such as fucosylated human milk oligosaccharides, glycoproteins, proteoglycans, glycosaminoglycans, lipopolysaccharides, capsular polysaccharides.

Thus, according to the invention, the nucleotide-activated sugar is GDP-fucose.

GDP-fucose is an essential component for the enzymatic synthesis of fucosylated oligosaccharides and other L-fucose containing glycoconjugates through glycosyltransferases. The chemical synthesis of GDP-fucose - which is widely used for the production of GDP-fucose today - is laborious and very inefficient. As an alternative, GDP-fuose ahs been generated via enzymatic in vitro-synthesis from GDP-D-mannose and NAD(P)H or from L-fucose, ATP, and GTP. Major disadvantages of these syntheses are, however, the availability of the needed substrates and co-factors needed, the synthesis of which are complex and costly. As a consequence, the main focus has been on the *in vivo*-production of this nucleotide-activated sugar in recombinant bacteria, which, however, has proven difficult due to the accumulation of GDP-fucose in the cytoplasm.

With the newly provided method, it is, for the first time, possible to transport the endogenously produced GDP-fucose across the membrane of the producing microorganism prokaryotic host cell, thus avoiding accumulation of GDP-fucose in the cytosol.

Accordingly, a "transporter protein for GDP-fucose" is presently understood to encompass and mean integral-membrane proteins that transport GDP-fucose from the cytoplasm across a membrane of said recombinant prokaryotic microorganism host cell. E.g., from eukaryotic cells transport proteins are known which transport, across a membrane, GDP-fucose from the cytoplasm in the Golgi.

The at least one membrane can be either the cytoplasmic membrane or the periplasmic membrane.

However, the use of such transporter proteins, in particular of eukaryotic transporter proteins, in prokaryotic microorganism cells, and their functional expression in prokaryotic microorganism cells has not been shown up to today. By using these transporters, an efficient transport of the intracellularly generated GDP-fucose across the e.g. cytoplasmic membrane is achieved, thus greatly increasing the overall production mass of GDP-fucose and highly facilitating its isolation/purification from the producing cells. Also, the provision of GDP-fucose in e.g. the periplasm of a recombinant prokaryotic microorganism host cell or in its medium of growth makes it possible to use said recombinant prokaryotic microorganism host cell for further processing GDP-fucose, e.g. by further genetically modifying said recombinant prokaryotic microorganism host cell.

Accordingly, and according to a preferred embodiment of the method according to the invention, said nucleic acid sequence heterologous to said recombinant prokaryotic microorganism cell and encoding a transporter protein for GDP-fucose is a nucleic acid sequence encoding an eukaryotic transporter protein being able to transport GDP-fucose from the cytosol across a membrane of said recombinant prokaryotic microorganism host cell, preferably into its periplasm and/or into the medium of its growth.

According to one aspect of the invention, the recombinant microorganism host cell used in the method according to the invention is transformed to comprise a nucleic acid sequence encoding a GDP-fucose transporter protein.

According to another aspect of the invention, the recombinant microorganism host cell used in the method according to the invention is transformed to comprise a nucleic acid sequence encoding a GDP-fucose transporter protein *from Homo sapiens.*

According to another aspect of the invention, a recombinant microorganism host cell is provided being transformed to comprise a nucleic acid sequence encoding a GDP-fucose transporter protein selected from the group consisting of the GDP-fucose transporter SLC35 C1 of *Homo sapiens,* the GDP-fucose transporter SLC35 C2 of *Homo sapiens* and the GDP-mannose/-fucose/-arabinose transporter of *Leishmania donovani* LPG2.

Also, according to a preferred embodiment, the prokaryotic microorganism host cell is selected from bacteria, and more preferably from gram-negative bacteria or gram-positive bacteria. According to preferred embodiments, the recombinant prokaryotic cell is a recombinant *Escherichia coli* cell, a recombinant *Bacillus sp.* cell, a recombinant *Corynebacterium* cell, a recombinant *Clostridium* cell. If a gram-negative bacterium is used, GDP-fucose may be retrieved from the periplasm or used by said host cell for further processing as discussed above.

Preferably, a recombinant prokaryotic microorganism host cell is used that does not endogenously express a GDP-L-fucose transporter.

According to yet another preferred embodiment, in the method according to the invention a recombinant prokaryotic microorganism host cell is used, wherein the nucleic acid sequence heterologous to said host cell and encoding the transporter protein for GDP-fucose comprises a sequence that is selected from:

a) SEQ-ID No. 1 of the enclosed sequence listing, b) a sequence encoding SEQ-ID No. 2 of the enclosed sequence listing, and c) nucleic acid sequences which hybridize under stringent conditions to the nucleic acid sequences defined in a) and b) or their complementary strands.

SEQ ID No. 1 of the enclosed sequence listing represents a synthetic nucleic acid having the codon usage of *Escherichia coli* and encoding the human GDP-fucose transporter gene SLC35 C1.

SEQ ID No. 2 of the enclosed sequence listing represents the amino acid sequence of the human GDP-fucose transporter gene SLC35 C1.

The expression "hybridizing specifically to", refers to the binding, duplexing, or hybridizing of a molecule only to a particular nucleotide sequence under stringent conditions when that sequence is present in a complex mixture (e.g., total cellular) DNA or RNA.

The term "stringent conditions" refers to conditions under which a probe will hybridize to its target subsequence, but to no other sequences. Stringent conditions are sequence-dependent and will be different in different circumstances. Longer sequences hybridize specifically at higher temperatures. Generally, stringent conditions are selected to be about 15 C lower than the thermal melting point (Tm) for the specific sequence at a defined ionic strength and pH. The Tm is the temperature (under defined ionic strength, pH, and nucleic acid concentration) at which 50% of the probes complementary to the target sequence hybridize to the target sequence at equilibrium. Exemplary stringent hybridization conditions can be as following: 50% formamide, 5x SSC, and 1 % SDS, incubating at 42 C, or, 5x SSC, 1 % SDS, incubating at 65 C, with wash in 0.2x SSC, and 0.1% SDS at 65 C.

According to an embodiment of the method according to the invention, GDP-fucose is isolated from the recombinant prokaryotic microorganism host cell, preferably from the periplasm of said recombinant prokaryotic microorganism host cell, or from the medium of its growth.

According to another embodiment of the method of the invention, GDP-fucose provided in step b) is provided for further use or processing in the periplasm of said recombinant prokaryotic microorganism host cell, or for further use or processing in the medium said recombinant prokaryotic microorganism is cultivated/ fermented in. With the first alternative, i.e. if GDP-fucose is provided for further use or processing in the periplasm, the provided GDP-fucose can be used by a glycosyltransferase present in the periplasm, which, in this case, is additionally recombinantly expressed by said host cell. Alternatively, e.g. glycosyltrasferase is added to the medium the recombinant prokaryotic host cell is cultivated in, and GDP-fucose is, consequently, used/processed in the medium by the glycosyltransferase in the medium. Thus, these embodiments of the invention can be used for the production of glycosylated oligosaccharides, in particular fucosylated oligosaccharides such as fucosylated human milk oligosaccharides, glycoproteins, proteoglycans, glycosaminoglycans, lipopolysaccharides, capsular polysaccharides, etc.

Generally, and throughout the present invention, the term "glycosyltransferase" designates and encompasses enzymes that are responsible for the biosynthesis of disaccharides, oligosaccharides and polysaccharides, and they catalyse the transfer of monosaccharide moieties from an activated nucleotide monosaccharide/sugar (the "glycosyl donor") to a glycosyl acceptor molecule. Non-limiting examples for glycosyltransferases which can be used according to the invention and which shall be part of the invention are, e.g., bacterial fucosyltransferases, and preferably an alpha-1,2-fucosyltransferase, and more preferably the alpha-1,2-fucosyltransferase encoded by the wbgL gene of E. coli:0126 (genebank acc. No. ADN43847).

Accordingly, the terms "alpha-1,2-fucosyltransferase" or "fucosyltransferase" or a nucleic acid/polynucleotide encoding an "alpha-1,2-fucosyltranferase" or "fucosyltransferase" refer to a glycosyltransferase that catalyzes the transfer of fucose from a donor substrate, for example, GDP-fucose, to an acceptor molecule in an alpha-1,2-linkage. The acceptor molecule can be a carbohydrate, an oligosaccharide, a protein or glycoprotein, or a lipid or glycolipid, and can be, e.g., N- acetylglucosamine, N-acetyllactosamine, galactose, fucose, sialic acid, glucose, lactose or any combination thereof.

Thus, according to another aspect of the method of the invention, GDP-fucose provided in step b) is provided for further use or processing in the periplasm or in the medium the recombinant prokaryotic microorganism host cell is cultivated in by a glycosyltransferase for the synthesis of a glycosylated protein, glycosylated lipid or a (glycosylated) oligosaccharide.

According to another aspect of the invention, the method according to the invention further comprises the step of c) further processing the GDP-fucose provided in step b) in the periplasm or the medium the recombinant prokaryotic microorganism host cell is cultivated in, by a glycosyltransferase present in the periplasm or in the medium, thus synthesizing a glycosylated protein, glycosylated lipid or glycosylated oligosaccharide.

Examples of glycosylated or sialylated oligosaccharides are, e.g. fucosylated oligosaccharides such as fucosylated human milk oligosaccharides, glycoproteins, proteoglycans, glycosaminoglycans, lipopolysaccharides, capsular polysaccharides, etc. Exemplary non-limiting human milk oligosaccharides include 3'-sialyllactose, 6'- sialyllactose, 3'-fucosyllactose, 2'-fucosyllactose, lacto-N-fucopentaose, lact-N-difucohexaose, lactodifucotetraose, and lacto-N-(neo)-tetraose, and many more.

Thus, and according to another aspect, e.g. lactose may be added to the medium, e.g. after production of GDP-fucose, for further use by a glycosyltransferase present either in the medium or in the periplasm (by recombinant expression through said host cell), together with the provided GDP-fucose.

According to one aspect of the method of the invention, step b) comprises adding of a monosaccharide, preferably fucose, to the medium the recombinant prokaryotic microorganism host cell is cultivated in.

By adding a certain monosaccharide to the recombinant prokaryotic host cell or rather to the medium of its growth, a desired nucleotide-activated monosaccharide can be produced, either by the host cell's endogenously expressed enzymes, or by using additionally genetically modified host microorganisms being able to produce a nucleotide-activated monosaccharide from the added non-nucleotide-activated monosaccharide.

E.g., fucose may be added, which is transported across the membrane of the prokaryotic microorganism, e.g. *E. coli,* into the cytosol. There, e.g. the enzymes fucose kinase and GDP-fucose phosphorylase generate GDP-fucose (salvage pathway). By applying a respectively modified prokaryotic microorganism host cell according to the invention, the GDP-fucose, can be transported from the cytosol across the membrane into either the periplasmic space/periplasm or into the medium of growth, thus providing for a continuous production of GDP-fucose. Alternatively, where applicable, the endogenous pathway (*de novo* pathway) of a prokaryotic microorganism, e.g. *E. coli,* may be utilized to produce GDP-fucose. In both cases/pathways, the recombinant prokaryotic microorganism can be (additionally) transformed to express the enzymes respectively involved in the pathways, and by overexpressing these enzymes an efficient production of GDP-fucose can be achived. Further, and in addition, enzymes involved in the degradation of products and/or intermediates might be inactivated. For example by using the fucose salvage pathway for the provision of GDP-Fucose, the fucose catabolic pathway should be inactivated. Similarly by using sialic acid as substrate, the sialic acid catabolic pathway should be inactivated or down regulated. This can be achieved by genomic inactivation of the catabolic pathway genes (i.e. knock-out) or by using antisense or similar techniques known in the field.

As already mentioned above, the invention also relates to a recombinant prokaryotic microorganism host cell to be stably cultured in a medium, the recombinant prokaryotic microorganism host cell being transformed to comprise at least one nucleic acid sequence heterologous to said recombinant prokaryotic microorganism host cell, wherein said nucleic acid heterologous to said recombinant prokaryotic microorganism host cell comprises a nucleic acid sequence encoding an eukaryotic transporter protein for a nucleotide-activated sugar that is fused to a signal sequence which transports said protein through a cellular membrane of the host cell, the eukaryotic transporter protein being able to transport the nucleotide-activated sugar from the cytosol across a membrane of said recombinant prokaryotic microorganism host cell, wherein the nucleotide-activated sugar is GDP-fucose.

As presently used, the expression "functionally expressed" is meant to define that the transporter protein heterologous to the microorganism cell is fulfilling its function it naturally has in it original source, i.e. the transport of GDP-fucose over cell membranes.

The term "prokaryotic microorganism host cell" as used herein includes bacteria, whereby preferred bacteria are Gram-negative or Gram-positive bacteria, preferably *Escherichia coli.*

The expression "cell" is also used for designating a culture of cells.

Also, in the method and the microorganism host cell according to the invention, it is preferred if the nucleic acid heterologous to the recombinant prokaryotic microorganism host cell and encoding the nucleotide-activated sugar transporter protein, e.g. the human GDP-fucose-transporter, is adapted to the codon usage of the respectively used recombinant prokaryotic microorganism cell.

Further, and according to one aspect of the invention, it is preferred if the microorganism host cell is selected from the group consisting of bacteria, and more preferably from gram-negative bacteria or gram-positive bacteria.

According to a preferred embodiment, the recombinant prokaryotic microorganism host cell is transformed to comprise and express a nucleic acid sequence encoding eukaryotic transporter protein being able to transport GDP-fucose from the cytosol of said host cell across a membrane of said host cell.

The features of said recombinant prokaryotic microorganism host cell as already explained above for the method according to the invention do also apply for the recombinant prokaryotic microorganism host cell as claimed hereinafter.

According to a preferred embodiment, the recombinant prokaryotic microorganism is transformed to comprise and express a nucleic acid sequence encoding a human nucleotide-activated sugar (preferably: hexose) transporter protein that is selected from the group consisting of the GDP-fucose transporter SLC35 C1 of *Homo sapiens,* the GDP-fucose transporter SLC35 C2 of *Homo sapiens* and the GDP-mannose/-fucose/- arabinose transporter of *Leishmania donovani* LPG2.

As already mentioned above for the method according to the invention using a recombinant prokaryotic microorganism host cell, and according to another aspect of the invention, it is preferred if the recombinant prokaryotic microorganism host cell has been transformed to comprise a nucleic acid sequence encoding an GDP-fucose-transporter comprising a sequence that is selected from: a) SEQ-ID No. 1 of the enclosed sequence listing, b) a sequence encoding SEQ-ID No. 2 of the enclosed sequence listing, and c) nucleic acid sequences which hybridize under stringent conditions to the nucleic acid sequences defined in a) and b) or their complementary strands.

It is to be understood that the prokaryotic recombinant microorganism host cell may be further modified to express, e.g. a glycosyltransferase as detailed above.

The disclosure also concerns a vector, containing a nucleic acid sequence heterologous to said cell, wherein said nucleic acid heterologous to said cell comprises a nucleic acid sequence encoding an eukaryotic transporter protein for a nucleotide-activated sugar, in particular a GDP-fucose-transporter of *Homo sapiens,* wherein the nucleic acid sequence is being operably linked to control sequences recognized by a microorganism host cell transformed with the vector, and as further detailed below.

The invention, accordingly, also concerns a recombinant prokaryotic microorganism host cell containing a vector according to the invention and as defined above and below.

For recombinant production, the microorganism host cells can be genetically engineered to incorporate expression systems, or portions thereof, for the nucleic acid encoding a transporter protein for a nucleotide-activated sugar Introduction of a heterologous nucleic acid into the host cell can be effected by methods described in many standard laboratory manuals, such as Davis et al., Basic Methods in Molecular Biology, (1986), and Sambrook et al., Molecular Cloning: A Laboratory Manual. Cold Spring Harbor Laboratory Press, 2nd edition (1989).

Thus, the nucleic acid sequence which is used according to the invention, may, e.g., be comprised in a vector which is to be stably transformed/transfected into prokaryotic microorganism host cells. In the vector, the nucleic acid sequence used according to the invention is under control of an, e.g., inducible promoter, so that the expression of the gene/polynucleotide can be specifically targeted, and, if desired, the gene may be overexpressed in that way.

A great variety of expression systems can be used to produce the polypeptides of the invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from bacterial genome, from viruses, and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook *et al., supra.*

In some embodiments, the nucleic acid sequence is placed under the control of an inducible promoter, which is a promoter that directs expression of a gene where the level of expression is alterable by environmental or developmental factors such as, for example, temperature, pH, anaerobic or aerobic conditions, light, transcription factors and chemicals. Such promoters are referred to herein as "inducible" promoters, which allow one to control the timing of expression of the transporter protein for GDP-fucose. For *E*. *coli* - and other bacterial host cells - inducible promoters are known to those of skill in the art.

According to one aspect, the nucleic acid sequence heterologous to said cell and comprising a nucleic acid sequence encoding a transporter protein for a nucleotide-activated sugar, in particular encoding the human GDP-fucose transporter, is fused to a signal sequence, in particular to a signal sequence which is selected from the groups consisting of OmpA, MaE, LamB, PelB, LivK, TorT, ToiB, DsbA, Pac, TorA, and PhoA. In alternative embodiments, the signal sequences can be a eukaryotic signal sequence.

The terms "signal sequence", "signal peptide" or "secretion signal sequence" as used herein refers to a stretch of amino acids within a polypeptide or protein which directs said polypeptide or protein, typically a newly synthesized polypeptide or protein, through a cellular membrane of a host cell. In prokaryotic cells, signal sequences typically direct polypeptides or proteins through the cytoplasmic membrane into the periplasmic space. Usually, the signal sequence is present at the N-terminus of a protein or polypeptide and facilitates its transport to the periplasm or into the culture medium of the host cell. Polypeptides and proteins comprising a signal sequence are referred to as "preprotein". The signal sequence is generally removed from the N-terminus of the preprotein by enzymatic cleavage during translocation through the membrane, thereby producing the mature protein.

The determination of signal sequences is well known to the person skilled in the art. For example, signal sequences can be obtained from databases such as Swiss-Prot or GenBank or using annotated genome- wide data sets.

Signal sequence of the present invention may be homologous or heterologous origin. A homologous signal sequence is derived from the same species as the polypeptide or protein to which it is fused. In contrast, a heterologous signal sequence is derived from a different species. Any homologous or heterologous signal sequence may be cloned in association with a polypeptide or protein which is to be transported through a cellular membrane or into the periplasmic space by the host cell.

All functional derivatives of the signal sequences and of the nucleic acid sequences encoding the transporter protein for the nucleotide-activated sugar can be used.

"Functional derivatives" as used in this context refers to any sequence which is based on any of the naturally occurring sequences, but which was modified, thereby still fulfilling its function (e.g. signal sequences: of directing polypeptides or proteins into the prokaryotic periplasmic space or through a cellular membrane; transporter protein sequences of transporting the specific compound). Modifications include introduced amino acid substitution, such as by site-directed mutagenesis of the respective nucleic acid encoding for said amino acids, and purposely introduced insertions or deletions.

The invention also relates to the use of the recombinant microorganism host cell as described above and below for producing GDP-fucose.

The invention further relates to the use of a sequence having SEQ ID No. 1 in the expression of the human GDP-fucose transporter in a microorganism host cell for producing GDP-fucose, which is why the invention also relates to GDP-fucose obtained by the method according to the invention, which can be used, e.g. for the production of fucosylated oligosaccharides.

Further advantages follow from the description of the embodiments and the attached drawings.

It goes without saying that the abovementioned features and the features which are still to be explained below can be used not only in the respectively specified combinations, but also in other combinations or on their own, without departing from the scope of the present invention.

### BRIEF DESCRIPTION OF THE FIGURES

Several embodiments of the invention are illustrated in the figures and explained in more detail in the following description. In the figures:

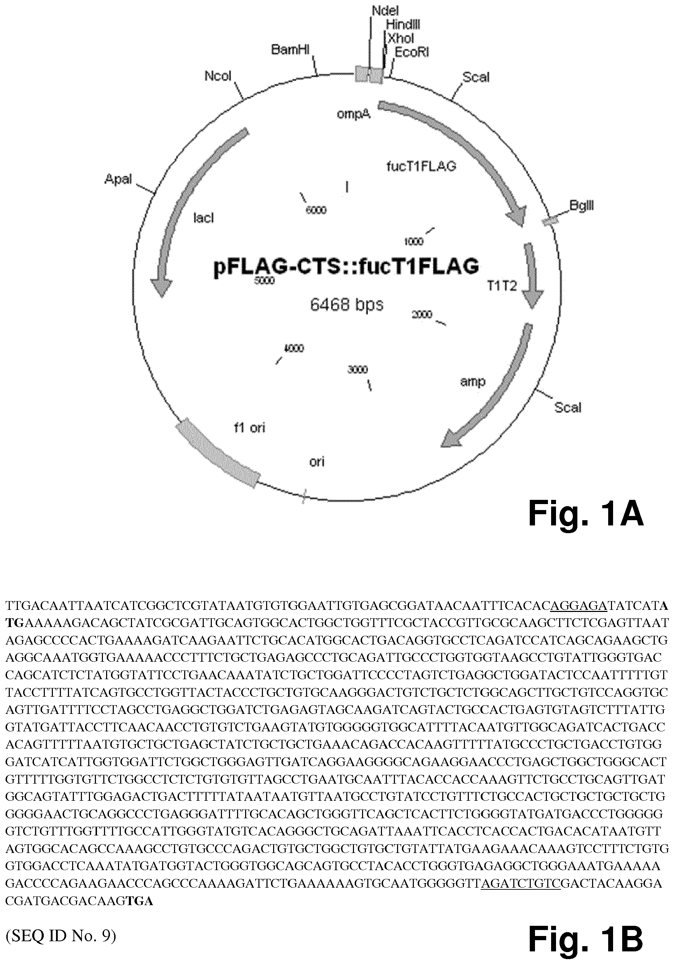

**Fig. 1** shows the map of plasmid pFLAG-CTS::*fucT1*FLAG, containing the synthetic *fucT1* gene, with the OmpA signal sequence of *E*. *coli* N-terminally fused to the *fucT1* gene, and with the FLAG-tag C-terminally fused to the *fucT1* gene **(****Fig. 1A****);** with the relevant sequences in **Fig. 1B****;**

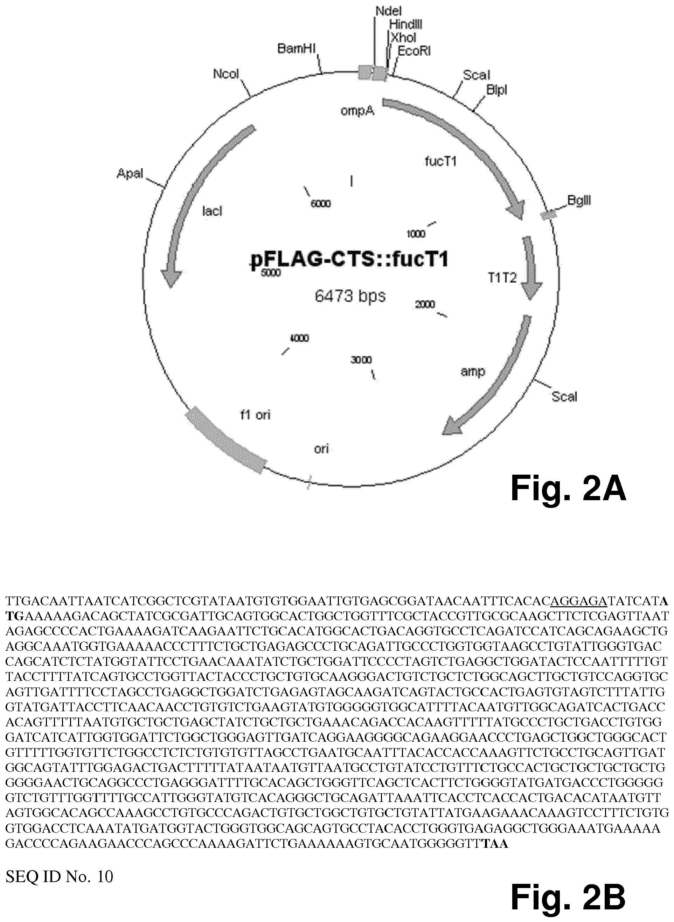

**Fig. 2** shows the map of plasmid pFLAG-CTS::fucT1, containing the synthetic *fucT1* gene, with the OmpA signal sequence of E. *coli* N-terminally fused to the *fucT1* gene, without the FLAG-tag C-terminally fused to the *fucT1* gene **(****Fig.2A****);** with the relevant sequences in **Fig. 2B****;**

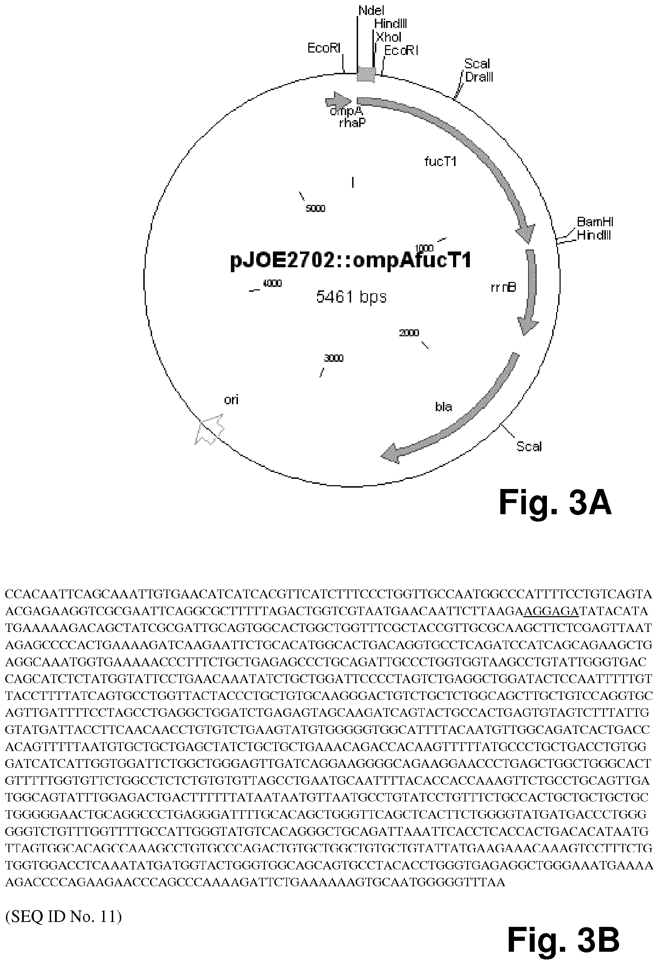

**Fig. 3** shows the map of plasmid *pJOE2702::ompAfucT1* **(****Fig. 3A****)** as well as the relevant sequences **(****Fig. 3B****);**

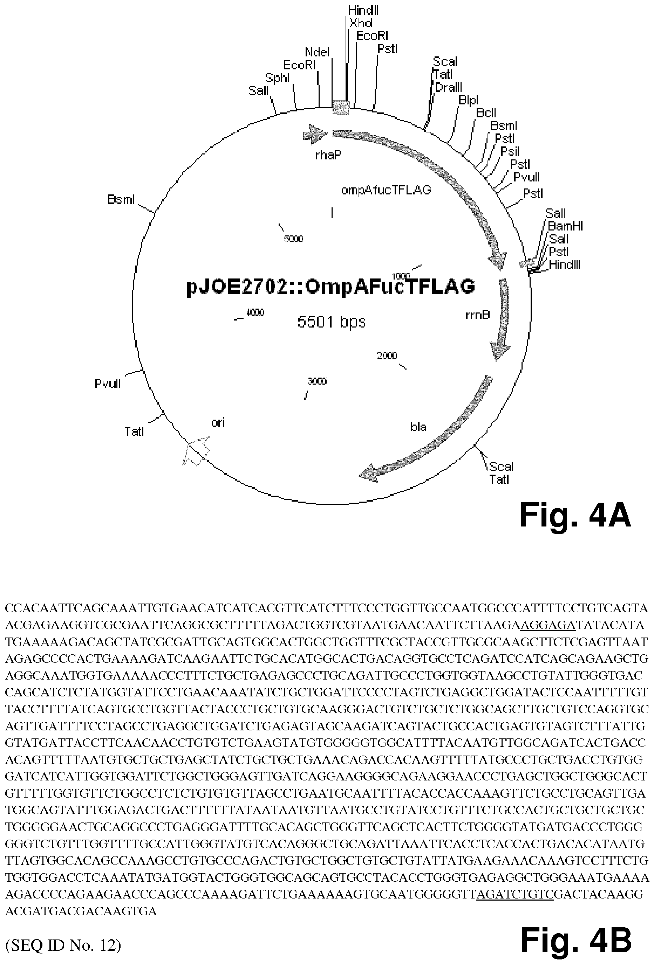

**Fig. 4** shows the map of plasmid pJOE *pJOE2702::ompAfucT1FLAG* **(****Fig. 4A****)** as well as the relevant sequences **(****Fig. 4B****);**

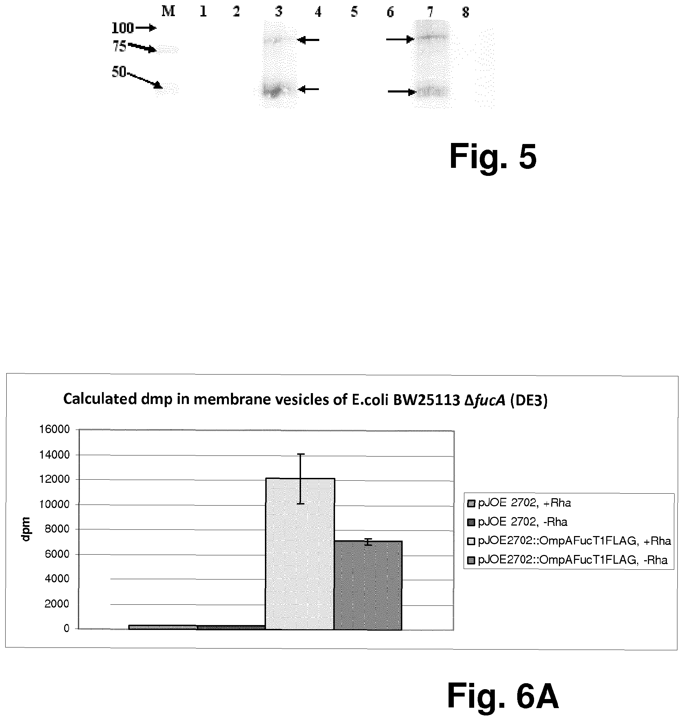

**Fig. 5** shows a Western Blot of solubilized membrane proteins of *E.coli* cell cultures transformed with either *pJOE2702::ompAfucT1* which have either been induced or not induced, or *pJOE2702::ompAfucT1FLAG* which have either been induced or not induced; also, solubilized membrane proteins of *E*. *coli* cell cultures transformed with control plasmids pJOE2702, induced and not-induced, are blotted; and

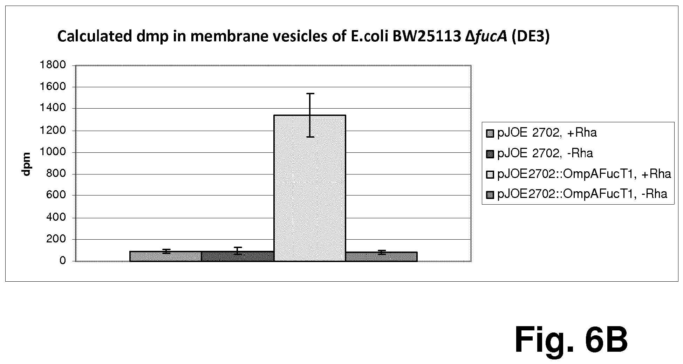

**Fig. 6** shows a diagram displaying the results for the uptake of radioactive GDP-fucose by membrane vesicles of *E*. *coli* BW25113 Δ *fucA* (DE3) with plasmid pJOE2702::*ompAfucTIFLAG* and with control plasmid pJOE2702, induced and not induced respectively **(****Fig. 6A****),** or of *E*. *coli* BW25113 Δ *fucA* (DE3) with plasmid *pJOE2702::ompAfucT1* and with control plasmid pJOE2702, induced and not induced respectively **(****Fig. 6B****).**

### DETAILLED DESCRIPTION OF THE FIGURES

In order to make available substances such as, e.g., GDP-L-fucose, which are produced by recombinant prokaryotic microorganisms, e.g. *Escherichia coli* strains (that do not have a GDP-L-fucose transporter), in their cytoplasm, transporter proteins are employed which are specific for these substances and which transport them out of the cytoplasm for further processing in or isolation from the periplasm of the cell or in the culture supernatant.

Thus, in an exemplary embodiment, and according to the invention, human gene SLC35 C1 (NCBI-Accession-Nr. NM018389.4) was selected for the GDP-fucose transport in *E*. *coli.* The DNA sequence of the human GDP-fucose transporter gene, *fucT,* was adapted *in silico* to the codon usage of *E*. *coli* (see SEQ ID No. 1). The resulting amino acid sequence (see SEQ ID No. 2) encoded by this DNA sequence was identical with the sequence of the human GDP-fucose transporter. The sequence of the human GDP-fucose transporter adapted to the codon usage of *E*. *coli* was synthesized, and cloned into vector pUC57, and two versions of the synthetic gene (see below) were recloned into vector pFLAG-CTS (Sigma-Aldrich). This vector contains the DNA sequence of the signal sequence of the OmpA protein of *E. coli* fused with the GDP-fucose transporter *fucT1* gene; further, by cloning this gene into the pFLAG-CTS vector, the *fucT1* gene comprised a C -terminal FLAG tag, thus obtaining vector pFLAG-*CTS::ompAfucT1FLAG.* The constructs were sequenced, and the correct DNA sequences and the correct reading frames confirmed.

In order to optimize expression, the construct consisting of the GDP-fucose transporter *fucT1* fused with the OmpA signal sequence and the C -terminal FLAG tag (*ompAfucTIFLAG*) was amplified from vector pFLAG-CTS::ompAfucT1FLAG and cloned into vector pJOE2702 (Altenbuchner, J. et al. (2000): "Ein neues, L-Rhamnose induzierbares Expressionssystem für Escherichia coli", Biospektrum 1/2000: 33-37). In this vector, the expression of genes is controlled by the L-rhamnose inducible rhapromoter. Alternatively, other well-regulated promoters such as the arabinose or tetracycline promoter can be used.

As mentioned above, the synthetic *fucT* gene was amplified in two versions, one comprising the stop codon using primers *fucT1Upper_Xho*I 5'- CCGCTCG AGTTAATAGAGCCCCACTGAAAAGATCAAG-3' (SEQ ID No. 3) and *fucT1Lower_Stop_BglII* 5'- GAAGATCTTCTTAAACCCCCATTGCACTTTTTTC-3' (SEQ ID No. 4), introducing a Xhol-restriction site or a Bg/ll-restriction site, and the other version without stop codon using primers *fucT1Upper_Xho*I 5'-CCGCTCGAGTTAATAGAG CCCCACTGAAAAGATCAAG-3' (SEQ ID No. 3) and *fucT1Lower_Bgl*II 5'-GAAGATCTAA CCCCCATTGCACTTTTTTCAG-3' (SEQ ID No. 5). Via the restriction sites *Xho*I and *Bgl*II the fragments were cloned into pFLAG-CTS. The fragment amplified without stop codon did not comprise the FLAG-tag. The resulting plasmids pFLAG-CTS::*fucT1*FLAG and pFLAG-CTS::*fuc*T1, as well as the relevant sequences contained therein, are shown in figures 1 and 2. The sequences (SEQ ID No. 9 and 10) shown, respectively, in Fig. 1B and 2B comprise the tac-promoter (position 1 to 73), fused to the OmpA signal sequence (74 to 145) fused to the *fucT1* gene (146 to 1234) and the FLAG-tag (1244 to 1267) (pFLAG-CTS::*fucT1*FLAG only, which also includes a spacer (1235 to 1243), followed by a stop-codon (TGA or TAA), respectively.

*fucT1* fused with the *ompA* signal sequence and the FLAG tag, or *fucT1* fused only with the *ompA* signal sequence were amplified from plasmids pFLAG-CTS::*fucT1*FLGA and pFLAG-CTS::*fucT1*, respectively, introducing *Nde*I and BamHl restriction sites, by using primers *fucT1_ompA_Nde*I 5'-GGAATTCCATATG AAAAAGACAGCTATCGCGATTG-3' (SEQ ID No. 6) and FLAG_Lower_*Bam*HI 5'-CGGGATCCCGGATCGATCTTCACTTGTCGTCATCG-3' (SE ID No. 7), or *fucT1Lower_Stop_BamH* 5'-CGGGATCCCGTTAAACCCCCATTGCACTTTTTTC-3' (SEQ ID No. 8). After restriction digestion the respective fragments were cloned into pJOE2702 via BamHI and *Nde*I, thereby generating plasmids *pJOE2702::ompAfucT1* and *pJOE2702::ompAfucT1FLAG.* The plasmids and the relevant DNA sequences are shown in Figures 3 and 4. The sequences (SEQ ID No. 11 and 12) shown, respectively, in Fig. 3B and 4B, comprise the rhamnose-promoter (position 1 to 147), fused to the OmpA signal sequence (position 148 to 219) fused to the *fucT1* gene (220 to 1310) and the FLAG-tag (1320 to 1343) (pJOE2702::*ompAfucT1FLAG* only, which also includes a spacer (1311 to 1319), followed by a stop-codon (TGA or TAA), respectively.

All methods were carried out according to standard methods (Sambrook et al., Molecular Cloning: A Laboratory Manual. Cold Spring Harbor Laboratory Press, 2nd edition (1989)).

Plasmids are listed in Table 1 below, and primers are shown in Table 2 below:

**Table 1: Plasmids and strains**

| **Plasmids** | **Characteristics** |
|---|---|
| pFLAG-CTS | *amp^{r}, lacI,* N-terminal *ompA-*signal sequence, FLAG-Tag, tac promoter |
| pFLAG-CTS::*fucT1* | pFLAG-CTS with GDP-fucose transporter gene *fucT1,* without C-terminal FLAG-Tag |
| pFLAG-CTS::*fuct1 FLAG* | pFLAG-CTS with GDP-fucose transporter gene *fucT1,* with C-terminal FLAG-tag |
| pJOE2702 | amp^{r}, Rhamnose-promoter |
| pJOE2702::*ompAfucT1* | pJOE2702 containing the GDP-fucose transporter gene *fucT1* fused with *ompA-*signal sequence |
| pJOE2702::*ompAfucT1FLAG* | pJOE2702 containing the GDP-fucose transporter gene *fucT1* fused with *ompA-*signal sequence and with FLAG-tag |

| **Strains** | |
|---|---|
| *E. coli* BW25113 Δ*fucA* (DE3) | modified strain *E*. *coli* BW25113 (Datsenko & Wanner, (2000): "One step inactivation of chromosomal genes in Escherichia coli K-12 using PCR products", PNAS. 97, 6640-66452000), deletion in *fucA,* carries gene for T7 RNA-polymerase, suitable for expression from the T7-promoter |

**Table 2: Primers**

| **Primer-Bezeichnung** | **DNA-Sequenz** | **SEQ ID No.** |
|---|---|---|
| *fucT1*Upper_*Xho*l | 5'-CGCTCGAGTTAATAGAGCCCCACTGAAAAGATCAAG-3' | 3 |
| *fucT1*Lower_Stop_*Bgl*ll | 5'-GAAGATCTTCTTAAACCCCCATTGCACTTTTTTC-3' | 4 |
| *fucT1*Lower_*Bgl*II | 5'-GAAGATCTAACCCCCATTGCACTTTTTTCAG-3' | 5 |
| *fucT1_ompA_Nde*I | 5'-GGAATTCCATATGAAAAAGACAGCTATCGCGATTG-3' | 6 |
| FLAG_Lower_*Bam*HI | 5'-CGGGATCCCGGATCGATCTTCACTTGTCGTCATCG-3' | 7 |
| *fucT1*Lower_Stop_*Bam*HI | 5-CGGGATCCCGTTAAACCCCCATTGCACTTTTTTC-3' | 8 |

| | | |
|---|---|---|
| Restriction sites underlined. | | |

### Detection of the expression and the localization of the GDP-fucose transporter protein

Via the FLAG tag it was possible to detect the expressed protein immunologically by Western blot. To check whether the GDP-fucose transporter protein was integrated into the membrane of *E. coli,* membranes were isolated from induced and non-induced cultures of *E. coli* BW25113 Δ *fucA* (DE3) pJOE2702:*:ompAfucTIFLAG* and from control cultures with plasmid pJOE2702: 400 ml of LB medium were inoculated with 100 µg/ml ampicillin in 2l Erlenmeyer flasks with an OD600 of 0.1 and incubated at 30°C in shake flasks culture. At an OD 600 of ∼ 0.6 (after ∼ 2.5 h), induction was effected with 0.01 % L-rhamnose. 5 hours after the induction the cells were harvested (Beckman Avanti J25 centrifuge, JA10 rotor, 5000 rpm , 15 min, 4° C) and stored until further use at -20°C (∼1 g wet weight per culture).

The proteins present in the membranes were treated with detergents in order to largely separate the membrane lipids. Two different solubilizing buffers were used: Buffer 1 contained 2.5 % SDS in 20 mM HEPES, pH 7.5, buffer 2 contained 2% sodium deoxycholate, 2% ASB 14-4, 2 M urea, 0.5 M thiourea and 2.5 % SDS in 20 mM HEPES, pH 7.5. Furthermore, two different methods were used for the separation of proteins from membrane lipids: In the first method, the cells were disrupted in one passage using a French press at 800 psi and 4°C. Debris was collected by centrifuging for 30 min at 13,000 x g, 4°C. The clear supernatant was subsequently centrifuged for 1 hour and 15 minutes at 80 000 x g in a TFT 65.13 rotor in a Beckman Optima LE 80 K centrifuge to separate the membranes. The thus obtained membranes were mixed with solubilizing buffer and incubated for 90 min on ice. The reaction mixture was vortexed every 5 min for 2-3 seconds to completely resuspend the membrane pellet. After centrifuging for 1 hour and 15 minutes at 80 000 × g in a TFT - 65.13 rotor in a Beckman Optima LE 80 K centrifuge, the membrane proteins could be found in the supernatant.

In the second method, membrane vesicles were mixed 1:1 with solubilizing buffer and incubated overnight at room temperature. The mixture was directly used for separation in the SDS gel. The solubilizing buffers used were prepared in accordance with Rehm, 2006 (Proteinbiochemie/Proteomics. Spektrum Akademischer Verlag, 5th edition).

The membrane proteins were separated in SDS -urea gel and subsequently blotted. An immunological detection was carried out, wherein the location of the GDP-fucose transporter protein in the membrane fraction was shown by detection of the FLAG- tag using anti-FLAG antibodies fused to alkaline phosphatase (AP). The AP fusion allows a simple coloring of the detected bands on the Western blot membrane. The Western blot was carried out according to Sambrook *et al.,* 1989, *supra.* Fig. 5 shows a Western blot, with the proteins in lanes 1-4 (7.5 µg per sample) dissolved with buffer 1, and the proteins in lanes 5-8 (5 µg per sample) dissolved with buffer 2 from the membrane of the membrane vesicles. In Fig. 5, the lanes were as follows:

M: protein standard (in kDa); lane 1: membrane proteins of *E. coli* BW25113 Δ *fucA* (DE3) pJOE2702, + L-Rhamnose; lane 2: membrane proteins of *E. coli* BW25113 Δ *fucA* (DE3) pJOE2702, - L-Rhamnose; lane 3: membrane proteins of *E. coli* BW25113 Δ *fucA* (DE3) pJOE2702::ompAfucT1FLAG, + L-Rhamnose; lane 4: membrane proteins of *E. coli* BW25113 Δ *fucA* (DE3) *pJOE2702::ompAfucT1FLAG,* - L-Rhamnose; lane 5: membrane proteins of *E. coli* BW25113 Δ *fucA* (DE3) pJOE2702, + L-Rhamnose; lane 6: membrane proteins of *E. coli* BW25113 Δ *fucA* (DE3) pJOE2702, - L-Rhamnose; lane 7: membrane proteins of *E. coli* BW25113 Δ *fucA* (DE3) JOE2702*::ompAfucT1FLAG,* + L-Rhamnose; lane 8: membrane proteins of *E. coli* BW25113 Δ *fucA* (DE3) pJOE2702::*ompAfuc*T1*FLAG,* - L-Rhamnose.

The protein detected below the 50 kDa marker band represented the FLAG -tagged monomer of the GDP-fucose transporter protein; the protein detected between the 75 kDa and 100 kDa marker band was the putative dimer of the FLAG - tagged GDP- fucose transporter protein.

### Detection of the GDP-fucose transport by the GDP-fucose transporter protein

To show an effective GDP-fucose transport via the GDP-fucose transporter protein, transport of ³H -labeled GDP-fucose was examined in inside-out membrane vesicles. The inside-out vesicles were obtained from cultures of *E. coli* BW25113 Δ *fucA* (DE3) pJOE2702::*ompAfucT1FLAG* and *E. coli* BW25113 Δ *fucA* (DE3) pJOE2702::*ompAfucT1*, either induced with 0.01% L-rhamnose or non-induced, and from control cultures with empty plasmid membranes according to the method of Rosen & Tsuchiya ("Preparation of everted membrane vesicles from Escherichia coli for the measurement of calcium transport", Meth. Enzymol. 56:233-241 (1979)): 400 ml of LB medium were inoculated with 100 µg/ml ampicillin in 2l-Erlenmeyer flasks with an OD600 of 0.1 and incubated at 30°C in shake flasks cultures. At an OD 600 of ∼ 0.6 (after ∼2.5 h), induction was effected with 0.01 % L-rhamnose. 5 hours after the induction, the cells were harvested (Beckman Avanti J25 centrifuge, JA10 rotor, 5000 rpm, 15 min, 4 ° C) (∼ 1 g wet weight per culture). The cells were resuspended in 1.5 ml of ice-cold 50 mM MOPS/KOH , pH 7.5 and disrupted in one passage using a French press at 800 psi and 4°C, thus generating inside-out membrane vesicles. The disruption was effected in presence of 10 mM GMP. Cell debris was collected by centrifuging for 30 min at 13,000 x g , 4°C. Subsequently, the clear supernatant was centrifuged for 1 hour and 15 minutes at 80 000 x g in a TFT 65.13 rotor in a Beckman Optima LE 80 K centrifuge to separate the membranes. The membranes were then washed and resuspended following a corresponding second centrifugation step. The membrane vesicles were checked by electron microscopy, and no differences could be detected between the membrane vesicles of the different cultures, which showed that the expression of the GDP-fucose transporter did not change the size or shape of the membrane vesicles.

In a next step, transporter assays in the membrane vesicles were performed using ³H-marked GDP-fucose. A transporter mixture (100 µl) contained 50 mM MOPS/KOH, pH 7.5, 50 µg protein and a) 2 220 000 dpm ³H-GDP-fucose (0.6 µM in the assay), or b) 222 000 dpm ³H-GDP-fucose and 50 nmol GDP-fucose (0.5 mM in the assay). Prior to the start of the assay, the components were incubated for 5 min at room temperature. The assay was started by adding ³H-GDP-fucose, or the mixture of ³H-GDP-fucose and GDP-fucose. After incubation of 10 min at room temperature, the assay was stopped by adding 100 µl ice-cold 50 mM MOPS/KOH, pH 7.5, 10 mM GMP (GMP was necessary for the transport). The assays were performed at 19°C. Immediately after this step, the membrane vesicles were separated by filtrating through nitrocellulose filters having a pore size of 0.025 µm and washed in two steps with 2 ml 50 mM MOPS/KOH, pH 7.5, and then with 3 ml 50 mM MOPS/KOH, pH 7.5. The filters were placed in scintillation vessels containing 3 ml Ultima Gold MV scintillation cocktail, and the radioactivity present in the membrane vesicles was measured in a scintillation counter.

An uptake of radioactivity could be shown for membrane vesicles derived from E. *coli* BW25113 Δ *fucA* (DE3) *pJOE2702::ompAfucT1FLAG* or from *E. coli* BW25113 Δ *fucA* (DE3) pJOE2702::*ompAfucT1.* No radioactivity uptake was observed for controls, i.e. in membrane vesicles of *E. coli* BW25113 Δ *fucA* (DE3) pJOE2702 and non-induced *E. coli* BW25113 Δ *fucA* (DE3) *pJOE2702::ompAfucT1FLAG* and *E. coli* BW25113 Δ *fucA* (DE3) pJOE2702::*ompAfucT1* cells. Figures 6A (plasmid *pJOE2702::ompAfucT1FLAG)* and 6B (plasmid pJOE2702::*ompAfucT1*) show representative transport assays measurements.

Transport kinetics were established, where, in an Eppendorf cup, the ³H-GDP-fucose solution was mixed with a corresponding amount of membrane vesicles mixed with 50 mM MOPS/KOH (50 µg protein). ³H-GDP-fucose concentrations of 0.58 µM to 5.8 µm were used. Mixing was performed by carefully and briefly pipetting. Also, prior to the assay, the components were incubated for 5 min at room temperature. The measured kinetic parameters were - when analyzed with a Lineweaver-Burk-Plot - at 6.57 µM and a vₘₐₓ of 6.82 pmol/mg^{∗}min. The analysis with a Michaelis-Menten-Plot resulted in a K_{M}-value of 4 µM and a vₘₐₓ of 8,02 pmol/mg^{∗}min.

These results show that a transport of GDP-fucose could be achieved with *E. coli* membranes comprising an integrated GDP-fucose transporter, showing that with the use of an nucleotide-activated sugar transporter in a microorganism that has been transformed with the respective transporter, the corresponding nucleotide-activated sugar can be produced.

## Claims

1. A method for producing GDP-fucose using a recombinant prokaryotic microorganism host cell, the recombinant prokaryotic host cell comprising a cytoplasm and at least one membrane, the method comprising the steps of:
a) providing a recombinant prokaryotic microorganism host cell, said recombinant prokaryotic microorganism host cell being transformed to comprise at least one nucleic acid sequence heterologous to said recombinant prokaryotic microorganism cell, said nucleic acid heterologous to said recombinant prokaryotic microorganism cell comprising a nucleic acid sequence encoding a transporter protein for GDP-fucose that is fused to a signal sequence which transports the protein through a cellular membrane of the host cell; and
b) growing, under suitable nutrient conditions permissive for the production of GDP-fucose, said recombinant prokaryotic host cell under conditions so as to allow said recombinant prokaryotic microorganism host cell to express said transporter protein for GDP-fucose, whereby the GDP-fucose is transported over the membrane; and thereby providing the GDP-fucose in the periplasm of said recombinant prokaryotic microorganism cell or the medium of its growth.

2. The method of claim 1, wherein the signal sequence is selected from the group of signal sequences consisting of OmpA, MaE, LamB, PelB, LivK, TotT, ToiB, DsbA, Pac, TorA and PhoA.

3. The method of claim 1 or 2, wherein said nucleic acid sequence heterologous to said recombinant prokaryotic microorganism cell and encoding a transporter protein for GDP-fucose is a nucleic acid sequence encoding an eukaryotic transporter protein being able to transport GDP-fucose from the cytosol across a membrane of said recombinant prokaryotic microorganism host cell, preferably into its periplasm and/or into the medium of its growth.

4. The method of any of claims 1 to 3, wherein a recombinant microorganism host cell is provided being transformed to comprise a nucleic acid sequence encoding a GDP-fucose transporter protein which is selected from the group consisting of the GDP-fucose transporter SLC35 C1 of *Homo sapiens,* the GDP-fucose transporter SLC35 C2 of *Homo sapiens* and the GDP-mannose/-fucose/- arabinose transporter of *Leishmania donovani* LPG2.

5. The method of any of the foregoing claims, wherein the prokaryotic microorganism host cell is selected from the group consisting of bacteria, preferably gram-negative and gram- positive bacteria.

6. The method of any of the forgoing claims, wherein the recombinant prokaryotic microorganism cell is selected from the group consisting of a recombinant *Escherichia coli* cell, a recombinant *Bacillus sp.* cell, a recombinant *Corynebacterium* cell, or a recombinant *Clostridium* cell.

7. The method of one of the foregoing claims, wherein the nucleic acid sequence encoding the transporter protein for GDP-fucose comprises a sequence that is selected from:
a) SEQ-ID No. 1
b) a sequence encoding SEQ-ID No. 2 or
c) nucleic acid sequences which hybridize under stringent conditions to the nucleic acid sequences defined in a) and b) or their complementary strands.

8. The method of any of the forgoing claims, wherein said GDP-fucose is isolated from the recombinant prokaryotic microorganism host cell or the medium of its growth.

9. The method of claim 8, wherein the GDP-fucose is isolated from the periplasm of said recombinant prokaryotic microorganism host cell.

10. The method of any of claims 1 to 7, wherein said GDP-fucose provided in step b) is provided for further use or processing in the periplasm or in the medium the recombinant prokaryotic microorganism host cell is cultivated in by a glycosyltransferase for the synthesis of a glycosylated protein, glycosylated lipid or an oligosaccharide.

11. The method of any of claims 1 to 7, further comprising the step of
c) further processing the GDP-fucose provided in step b) in the periplasm or the medium the recombinant prokaryotic microorganism host cell is cultivated in, by a glycosyltransferase present in the periplasm or in the medium, thus synthesizing a glycosylated protein, glycosylated lipid or an oligosaccharide.

12. The method of any of claims 1 to 11, wherein step b) comprises adding the monosaccharide fucose to the medium the recombinant prokaryotic microorganism host cell is cultivated in.

13. A recombinant prokaryotic microorganism host cell to be stably cultured in a medium, the recombinant prokaryotic microorganism host cell being transformed to comprise at least one nucleic acid sequence heterologous to said recombinant prokaryotic microorganism host cell, **characterized in that** said nucleic acid heterologous to said recombinant prokaryotic microorganism host cell comprises a nucleic acid sequence encoding an eukaryotic transporter protein for a nucleotide-activated sugar that is fused to a signal sequence which transports said protein through a cellular membrane of the host cell, the eukaryotic transporter protein being able to transport the nucleotide-activated sugar from the cytosol across a membrane of said recombinant prokaryotic microorganism host cell, wherein the nucleotide-activated sugar is GDP-fucose.

14. The recombinant prokaryotic microorganism host cell according to claim 13, **characterized in that** the nucleic acid sequence encoding a nucleotide-activated sugar-transporter is selected from GDP-fucose transporter SLC35 C1 of *Homo sapiens,* the GDP-fucose transporter SLC35 C2 of *Homo sapiens,* the GDP-mannose/-fucose/- arabinose transporter of *Leishmania donovani* LPG2.

15. The recombinant prokaryotic microorganism host cell of claim 13 or 14, wherein said recombinant prokaryotic microorganism host cell is selected from the group consisting of gram-negative bacterium cells, preferably *Escherichia coli.*

16. The recombinant prokaryotic host cell of any of claims 13 to 15, wherein the nucleic acid sequence encoding the nucleotide-activated sugar-transporter comprises a sequence that is selected from:
a) SEQ-ID No. 1
b) a sequence encoding SEQ-ID No. 2 (human GDP-fucose transporter) and
c) nucleic acid sequences which hybridize under stringent conditions to the nucleic acid sequences defined in a) and b) or their complementary strands.

17. Use of the recombinant prokaryotic microorganism host cell according to any of claims 13 to 16 for producing GDP-fucose.

18. Use of a sequence having SEQ ID No. 1 in the expression of the GDP-fucose transporter in a prokaryotic microorganism host cell for producing GDP-fucose in said prokaryotic microorganism host cell, wherein said GDP-fucose transporter is fused to a signal sequence which transports said protein through a cellular membrane of the host cell.

## Patentansprüche

1. Ein Verfahren zur Herstellung von GDP-Fucose unter Verwendung einer rekombinanten prokaryotischen Mikroorganismen-Wirtszelle, die rekombinante prokaryotische Mikroorganismen-Wirtszelle weist ein Zellplasma und mindestens eine Membran auf, das Verfahren umfasst die Schritte:
a) Bereitstellen einer rekombinanten prokaryotischen Mikroorganismen-Wirtszelle, die rekombinante prokaryotische Mikroorganismen-Wirtszelle ist transformiert, um mindestens eine für die rekombinante prokaryotische Mikroorganismen-Wirtszelle heterologe Nukleinsäuresequenz aufzuweisen, die für die rekombinante prokaryotische Mikroorganismen-Wirtszelle heterologe Nukleinsäuresequenz weist eine Nukleinsäuresequenz auf, die ein Transportprotein für GDP-Fucose kodiert, das mit einer Signalsequenz fusioniert ist, die das Protein durch eine zelluläre Membran der Wirtszelle transportiert; und
b) Wachsen, unter geeigneten Nährstoffbedingungen die die Herstellung von GDP-Fucose erlauben, der rekombinanten prokaryotischen Wirtszelle unter Bedingungen die es der rekombinanten prokaryotischen Mikroorganismen-Wirtszelle ermöglichen, das Transportprotein für GDP-Fucose zu exprimieren, wodurch die GDP-Fucose durch die Membran transportiert wird; und dadurch GDP-Fucose im Periplasma der rekombinanten prokaryotischen Mikroorganismen-Wirtszelle oder ihrem Wachstumsmedium bereitgestellt wird.

2. Das Verfahren nach Anspruch 1, wobei die Signalsequenz aus der Gruppe von Signalsequenzen ausgewählt ist, die aus OmpA, MaE, LamB, PelB, LivK, TotT, ToiB, DsbA, Pac, TorA und PhoA besteht.

3. Das Verfahren nach Anspruch 1 oder 2, wobei die für die rekombinante prokaryotische Mikroorganismen-Zelle heterologe und ein Transportprotein für GDP-Fucose kodierende Nukleinsäuresequenz eine Nukleinsäuresequenz ist, die für ein eukaryotisches Transportprotein kodiert, das GDP-Fucose aus dem Zytosol durch eine Membran der rekombinanten prokaryotischen Mikroorganismen-Wirtszelle transportieren kann, vorzugsweise in ihr Periplasma und/oder in ihr Wachstumsmedium.

4. Das Verfahren nach einem der Ansprüche 1 bis 3, wobei eine rekombinante Mikroorgansimen-Wirtszelle bereitgestellt wird, die transformiert ist, um eine Nukleinsäuresequenz aufzuweisen, die ein GDP-Fucose-Transportprotein kodiert, das aus der Gruppe ausgewählt ist, die aus dem GDP-Fucose-Transporter SLC35 C1 aus *Homo sapiens,* dem GDP-Fucose-Transporter SLC35 C2 aus *Homo sapiens* und dem GDP-Mannose/-Fucose/-Arabinose-Transporter aus *Leishmania donovani* LPG2 besteht.

5. Das Verfahren nach einem der vorhergehenden Ansprüche, wobei die prokaryotische Mikroorganismen-Wirtszelle aus der Gruppe ausgewählt ist, die aus Bakterien besteht, vorzugsweise Gram-negativen und Gram-positiven Bakterien.

6. Das Verfahren nach einem der vorhergehenden Ansprüche, wobei die rekombinante prokaryotische Mikroorganismen-Zelle aus der Gruppe ausgewählt ist, die aus einer rekombinanten *Escherichia coli*-Zelle, einer rekombinanten *Bacillus sp.*-Zelle, einer rekombinanten *Corynebacterium-Zelle* oder einer rekombinanten *Clostridium*-Zelle besteht.

7. Das Verfahren nach einem der vorhergehenden Ansprüche, wobei die das Transportprotein für GDP-Fucose kodierende Nukleinsäuresequenz eine Sequenz aufweist, die ausgewählt ist aus:
a) SEQ-ID No. 1,
b) einer Sequenz die SEQ-ID No. 2 kodiert, oder
c) Nukleinsäuresequenzen die unter stringenten Bedingungen mit den in a) und b) definierten Nukleinsäuresequenzen oder deren komplementären Strängen hybridisiert.

8. Das Verfahren nach einem der vorhergehenden Ansprüche, wobei die GDP-Fucose aus der rekombinanten prokaryotischen Mikroorganismen-Wirtszelle oder dem Medium isoliert wird, in dem sie gewachsen ist.

9. Das Verfahren nach Anspruch 8, wobei die GDP-Fucose aus dem Periplasma der rekombinanten prokaryotischen Mikroorganismen-Wirtszelle isoliert wird.

10. Das Verfahren nach einem der Ansprüche 1 bis 7, wobei die in Schritt b) bereitgestellte GDP-Fucose für eine weitere Verwendung oder Verarbeitung im Periplasma oder in dem Medium, in dem die rekombinante prokaryotische Mikroorganismen-Wirtszelle kultiviert wird, durch eine Glycosyltransferase für die Synthese eines glycosylierten Proteins, glycosylierten Lipids oder eines Oligosaccharids bereitgestellt wird.

11. Das Verfahren nach einem der Ansprüche 1 bis 7, zusätzlich aufweisend den Schritt
c) zusätzliches Weiterverarbeiten der in Schritt b) bereitgestellten GDP-Fucose im Periplasma oder in dem Medium, in dem die rekombinante prokaryotische Mikroorganismen-Wirtszelle kultiviert wird, durch eine Glycosyltransferase, die im Periplasma oder in dem Medium vorhanden ist, dadurch Synthetisieren eines glycosylierten Proteins, glycosylierten Lipids oder eines Oligosaccharids.

12. Das Verfahren nach einem der Ansprüche 1 bis 11, wobei Schritt b) das Zugeben des Monosaccharids Fucose zu dem Medium, in dem die rekombinante prokaryotische Mikroorganismen-Wirtszelle kultiviert wird, umfasst.

13. Eine rekombinante prokaryotische Mikroorganismen-Wirtszelle zur stabilen Kultivierung in einem Medium, die rekombinante prokaryotische Mikroorganismen-Wirtszelle ist transformiert, um mindestens eine für die rekombinante prokaryotische Mikroorganismen-Wirtszelle heterologe Nukleinsäuresequenz aufzuweisen, **dadurch gekennzeichnet, dass** die für die rekombinante prokaryotische Mikroorganismen-Wirtszelle heterologe Nukleinsäuresequenz eine Nukleinsäuresequenz aufweist, die ein eukaryotisches Transportprotein für einen Nukleotid-aktivierten Zucker kodiert, das mit einer Signalsequenz fusioniert ist, die das Protein durch eine zelluläre Membran der Wirtszelle transportiert, das eukaryotische Transportprotein kann den Nukleotid-aktivierten Zucker vom Zytosol durch eine Membran der rekombinanten prokaryotischen Mikroorganismen-Wirtszelle transportieren, wobei der Nukleotidaktivierte Zucker GDP-Fucose ist.

14. Die rekombinante prokaryotische Mikroorganismen-Wirtszelle gemäß Anspruch 13, **dadurch gekennzeichnet, dass** die den Transporter für den Nukleotid-aktivierten Zucker kodierende Nukleinsäuresequenz ausgewählt ist aus GDP-Fucose-Transporter SLC35 C1 aus *Homo sapiens,* dem GDP-Fucose-Transporter SLC35 C2 von *Homo sapiens,* dem GDP-Mannose/-Fucose/-Arabinose-Transporter von *Leishmania donovani* LPG2.

15. Die rekombinante prokaryotische Mikroorganismen-Wirtszelle nach Anspruch 13 oder 14, wobei die rekombinante prokaryotische Mikroorganismen-Wirtszelle aus der Gruppe ausgewählt ist, die aus Gram-negativen Bakterienzellen besteht, vorzugsweise *Escherichia coli.*

16. Die rekombinante prokaryotische Mikroorganismen-Wirtszelle nach einem der Ansprüche 13 bis 15, wobei die den Transporter für den Nukleotid-aktivierten Zucker kodierende Nukleinsäuresequenz eine Sequenz aufweist, die ausgewählt ist aus:
a) SEQ-ID No. 1,
b) einer Sequenz, die SEQ-ID No. 2 (humaner GDP-Fucose Transporter) kodiert, und
c) Nukleinsäuresequenzen die unter stringenten Bedingungen mit den definiert in a) und b) Nukleinsäuresequenzen oder deren komplementären Strängen hybridisieren.

17. Verwendung der rekombinanten prokaryotischen Mikroorganismen-Wirtszelle gemäß einem der Ansprüche 13 bis 16 zur Herstellung von GDP-Fucose.

18. Verwendung einer Sequenz, die die SEQ ID No. 1 hat, bei der Expression des GDP-Fucose-Transporters in einer prokaryotischen Mikroorganismen-Wirtszelle zur Herstellung von GDP-Fucose durch die prokaryotische Mikroorganismen-Wirtszelle, wobei der GDP-Fucose-Transporter mit einer Signalsequenz fusioniert ist, die das Protein durch eine zelluläre Membran der Wirtszelle transportiert.

## Revendications

1. Méthode de production de GDP-fucose en utilisant une cellule hôte de microorganisme procaryote recombinée, la cellule hôte procaryote recombinée comprenant un cytoplasme et au moins une membrane, la méthode comprenant les étapes consistant à :
a) mettre à disposition une cellule hôte de microorganisme procaryote recombinée, ladite cellule hôte de microorganisme procaryote recombinée étant transformée afin de comprendre au moins une séquence d'acide nucléique hétérologue vis-à-vis de ladite cellule de microorganisme procaryote recombinée, ledit acide nucléique hétérologue vis-à-vis de ladite cellule de microorganisme procaryote recombinée comprenant une séquence d'acide nucléique codant pour une protéine transporteuse pour le GDP-fucose qui est fusionnée avec une séquence signal qui transporte la protéine à travers une membrane cellulaire de la cellule hôte ; et
b) cultiver, dans des conditions convenables de nutriments permettant la production de GDP-glucose, ladite cellule hôte procaryote recombinée dans des conditions telles que permettant à ladite cellule hôte de microorganisme procaryote recombinée d'exprimer ladite protéine transporteuse pour le GDP-fucose, ce par quoi le GDP-fucose est transporté à travers la membrane ; et
fournissant ainsi le GDP-fucose dans le périplasme de ladite cellule de microorganisme procaryote recombinée ou le milieu de sa croissance.

2. Méthode selon la revendication 1, dans laquelle la séquence signal est choisie dans le groupe des séquences signal constitué par OmpA, MaE, LamB, PelB, LivK, TotT, ToiB, DsbA, Pac, TorA et PhoA.

3. Méthode selon la revendication 1 ou 2, dans laquelle ladite séquence d'acide nucléique hétérologue vis-à-vis de ladite cellule de microorganisme procaryote recombinée et codant pour une protéine transporteuse pour le GDP-fucose est une séquence d'acide nucléique codant pour une protéine transporteuse eucaryote capable de transporter du GDP-fucose à partir du cytosol à travers une membrane de ladite cellule hôte de microorganisme procaryote recombinée, préférablement dans son périplasme et/ou dans le milieu de sa croissance.

4. Méthode selon l'une quelconque des revendications 1 à 3, dans laquelle on met à disposition une cellule hôte de microorganisme recombinée, qui est transformée afin de comprendre une séquence d'acide nucléique codant pour une protéine transporteuse de GDP-fucose, qui est choisie dans le groupe constitué par le transporteur de GDP-fucose SLC35 C1 *d'Homo sapiens,* le transporteur de GDP-fucose SLC35 C2 *d'Homo sapiens,* et le transporteur de GDP-mannose/fucose/arabinose de *Leishmania donovani* LPG2.

5. Méthode selon l'une quelconque des revendications précédentes, dans laquelle la cellule hôte de microorganisme procaryote est choisie dans le groupe constitué par les bactéries, préférablement les bactéries à Gram négatif et à Gram positif.

6. Méthode selon l'une quelconque des revendications précédentes, dans laquelle la cellule de microorganisme procaryote recombinée est choisie dans le groupe constitué par une cellule d'*Escherichia coli* recombinée, une cellule de *Bacillus sp.* recombinée, une cellule de *Corynebacterium* recombinée, ou une cellule de *Clostridium* recombinée.

7. Méthode selon l'une des revendications précédentes, dans laquelle la séquence d'acide nucléique codant pour la protéine transporteuse pour le GDP-fucose comprend une séquence qui est choisie parmi :
a) SEQ ID n° 1 ;
b) une séquence codant pour SEQ ID n° 2 ; ou
c) des séquences d'acide nucléique qui s'hybrident, dans des conditions stringentes, avec les séquences d'acide nucléique définies dans a) et b) ou leurs brins complémentaires.

8. Méthode selon l'une quelconque des revendications précédentes, dans laquelle ledit GDP-fucose est isolé à partir de la cellule hôte de microorganisme procaryote recombinée ou du milieu de sa croissance.

9. Méthode selon la revendication 8, dans laquelle le GDP-fucose est isolé à partir du périplasme de ladite cellule hôte de microorganisme procaryote recombinée.

10. Méthode selon l'une quelconque des revendications 1 à 7, dans laquelle ledit GDP-fucose fourni dans l'étape b) est fourni pour une utilisation ou un traitement supplémentaire dans le périplasme ou dans le milieu dans lequel la cellule hôte de microorganisme procaryote recombinée est cultivée, par une glycosyltransférase pour la synthèse d'une protéine glycosylée, d'un lipide glycosylé ou d'un oligosaccharide.

11. Méthode selon l'une quelconque des revendications 1 à 7, comprenant en outre l'étape consistant à :
c) traiter davantage le GDP-fucose fourni dans l'étape b) dans le périplasme ou le milieu dans lequel la cellule hôte de microorganisme procaryote recombinée est cultivée, par une glycosyltransférase présente dans le périplasme ou dans le milieu, synthétisant ainsi une protéine glycosylée, un lipide glycosylé ou un oligosaccharide.

12. Méthode selon l'une quelconque des revendications 1 à 11, dans laquelle l'étape b) comprend l'addition du monosaccharide, le fucose, au milieu dans lequel la cellule hôte de microorganisme procaryote recombinée est cultivée.

13. Cellule hôte de microorganisme procaryote recombinée devant être cultivée de manière stable dans un milieu, la cellule hôte de microorganisme procaryote recombinée étant transformée afin de comprendre au moins une séquence d'acide nucléique hétérologue vis-à-vis de ladite cellule hôte de microorganisme procaryote recombinée, **caractérisé en ce que** ledit acide nucléique hétérologue vis-à-vis de ladite cellule de microorganisme procaryote recombinée comprenant une séquence d'acide nucléique codant pour une protéine transporteuse eucaryote pour un sucre à activation nucléotidique, qui est fusionnée avec une séquence signal qui transporte ladite protéine à travers une membrane cellulaire de la cellule hôte, la protéine transporteuse eucaryote étant capable de transporter le sucre à activation nucléotidique à partir du cytosol à travers une membrane de ladite cellule hôte de microorganisme procaryote recombinée, où le sucre à activation nucléotidique est le GDP-fucose.

14. Cellule hôte de microorganisme procaryote recombinée selon la revendication 13, **caractérisée en ce que** la séquence d'acide nucléique codant pour une protéine transporteuse de sucre à activation nucléotidique est choisie dans le groupe constitué par le transporteur de GDP-fucose SLC35 C1 *d'Homo sapiens,* le transporteur de GDP-fucose SLC35 C2 *d'Homo sapiens,* et le transporteur de GDP-mannose/fucose/arabinose de *Leishmania donovani* LPG2.

15. Cellule hôte de microorganisme procaryote recombinée selon la revendication 13 ou 14, ladite cellule hôte de microorganisme procaryote recombinée étant choisie dans le groupe constitué par les bactéries à Gram négatif, préférablement *Escherichia coli.*

16. Cellule hôte procaryote recombinée selon l'une quelconque des revendications 13 à 15, dans laquelle la séquence d'acide nucléique codant pour le transporteur de sucre à activation nucléotidique comprend une séquence qui est choisie parmi :
a) SEQ ID n° 1 ;
b) une séquence codant pour SEQ ID n° 2 (transporteur de GDP-fucose humain) ; et
c) des séquences d'acide nucléique qui s'hybrident, dans des conditions stringentes, avec les séquences d'acide nucléique définies dans a) et b) ou leurs brins complémentaires.

17. Utilisation de la cellule hôte de microorganisme procaryote recombinée selon l'une quelconque des revendications 13 à 16, pour la production de GDP-fucose.

18. Utilisation d'une séquence répondant à SEQ ID n° 1, dans l'expression du transporteur de GDP-fucose dans une cellule hôte de microorganisme procaryote, afin de produire du GDP-fucose dans ladite cellule hôte de microorganisme procaryote, où ledit transporteur de GDP-fucose est fusionné avec une séquence signal qui transporte ladite protéine à travers une membrane cellulaire de la cellule hôte.


### SEQUENCE LISTING

<110> Jennewein Biotechnologie GmbH
<120> Method for producing nucleotide-activated sugars and recombinant prokaryotic microorganism host cells used therefor
<130> 2827P101EP
<160> 12
<170> PatentIn version 3.5
<210> 1
   <211> 1095
   <212> DNA
   <213> Artificial Sequence
<220>
   <223> synthetic FucT1 gene
<400> 1
   atgaatagag ccccactgaa aagatcaaga attctgcaca tggcactgac aggtgcctca      60
   gatccatcag cagaagctga ggcaaatggt gaaaaaccct ttctgctgag agccctgcag     120
   attgccctgg tggtaagcct gtattgggtg accagcatct ctatggtatt cctgaacaaa     180
   tatctgctgg attcccctag tctgaggctg gatactccaa tttttgttac cttttatcag     240
   tgcctggtta ctaccctgct gtgcaaggga ctgtctgctc tggcagcttg ctgtccaggt     300
   gcagttgatt ttcctagcct gaggctggat ctgagagtag caagatcagt actgccactg     360
   agtgtagtct ttattggtat gattaccttc aacaacctgt gtctgaagta tgtgggggtg     420
   gcattttaca atgttggcag atcactgacc acagttttta atgtgctgct gagctatctg     480
   ctgctgaaac agaccacaag tttttatgcc ctgctgacct gtgggatcat cattggtgga     540
   ttctggctgg gagttgatca ggaaggggca gaaggaaccc tgagctggct gggcactgtt     600
   tttggtgttc tggcctctct gtgtgttagc ctgaatgcaa tttacaccac caaagttctg     660
   cctgcagttg atggcagtat ttggagactg actttttata ataatgttaa tgcctgtatc     720
   ctgtttctgc cactgctgct gctgctgggg gaactgcagg ccctgaggga ttttgcacag     780
   ctgggttcag ctcacttctg gggtatgatg accctggggg gtctgtttgg ttttgccatt     840
   gggtatgtca cagggctgca gattaaattc acctcaccac tgacacataa tgttagtggc     900
   acagccaaag cctgtgccca gactgtgctg gctgtgctgt attatgaaga aacaaagtcc     960
   tttctgtggt ggacctcaaa tatgatggta ctgggtggca gcagtgccta cacctgggtg    1020
   agaggctggg aaatgaaaaa gaccccagaa gaacccagcc caaaagattc tgaaaaaagt    1080
   gcaatggggg tttaa                                                      1095
<210> 2
   <211> 364
   <212> PRT
   <213> Homo sapiens
<400> 2
<210> 3
   <211> 36
   <212> DNA
   <213> Artificial Sequence
<220>
   <223> Primer
<400> 3
   cgctcgagtt aatagagccc cactgaaaag atcaag 36
<210> 4
   <211> 34
   <212> DNA
   <213> Artificial Sequence
<220>
   <223> Primer
<400> 4
   gaagatcttc ttaaaccccc attgcacttt tttc 34
<210> 5
   <211> 31
   <212> DNA
   <213> Artificial Sequence
<220>
   <223> Primer
<400> 5
   gaagatctaa cccccattgc acttttttca g 31
<210> 6
   <211> 35
   <212> DNA
   <213> Artificial Sequence
<220>
   <223> Primer
<400> 6
   ggaattccat atgaaaaaga cagctatcgc gattg 35
<210> 7
   <211> 35
   <212> DNA
   <213> Artificial Sequence
<220>
   <223> Primer
<400> 7
   cgggatcccg gatcgatctt cacttgtcgt catcg 35
<210> 8
   <211> 34
   <212> DNA
   <213> Artificial Sequence
<220>
   <223> Primer
<400> 8
   cgggatcccg ttaaaccccc attgcacttt tttc 34
<210> 9
   <211> 1270
   <212> DNA
   <213> Artificial Sequence
<220>
   <223> vector sequences (partial)
<400> 9
   ttgacaatta atcatcggct cgtataatgt gtggaattgt gagcggataa caatttcaca      60
   caggagatat catatgaaaa agacagctat cgcgattgca gtggcactgg ctggtttcgc     120
   taccgttgcg caagcttctc gagttaatag agccccactg aaaagatcaa gaattctgca     180
   catggcactg acaggtgcct cagatccatc agcagaagct gaggcaaatg gtgaaaaacc     240
   ctttctgctg agagccctgc agattgccct ggtggtaagc ctgtattggg tgaccagcat     300
   ctctatggta ttcctgaaca aatatctgct ggattcccct agtctgaggc tggatactcc     360
   aatttttgtt accttttatc agtgcctggt tactaccctg ctgtgcaagg gactgtctgc     420
   tctggcagct tgctgtccag gtgcagttga ttttcctagc ctgaggctgg atctgagagt     480
   agcaagatca gtactgccac tgagtgtagt ctttattggt atgattacct tcaacaacct     540
   gtgtctgaag tatgtggggg tggcatttta caatgttggc agatcactga ccacagtttt     600
   taatgtgctg ctgagctatc tgctgctgaa acagaccaca agtttttatg ccctgctgac     660
   ctgtgggatc atcattggtg gattctggct gggagttgat caggaagggg cagaaggaac     720
   cctgagctgg ctgggcactg tttttggtgt tctggcctct ctgtgtgtta gcctgaatgc     780
   aatttacacc accaaagttc tgcctgcagt tgatggcagt atttggagac tgacttttta     840
   taataatgtt aatgcctgta tcctgtttct gccactgctg ctgctgctgg gggaactgca     900
   ggccctgagg gattttgcac agctgggttc agctcacttc tggggtatga tgaccctggg     960
   gggtctgttt ggttttgcca ttgggtatgt cacagggctg cagattaaat tcacctcacc    1020
   actgacacat aatgttagtg gcacagccaa agcctgtgcc cagactgtgc tggctgtgct    1080
   gtattatgaa gaaacaaagt cctttctgtg gtggacctca aatatgatgg tactgggtgg    1140
   cagcagtgcc tacacctggg tgagaggctg ggaaatgaaa aagaccccag aagaacccag    1200
   cccaaaagat tctgaaaaaa gtgcaatggg ggttagatct gtcgactaca aggacgatga    1260
   cgacaagtga                                                           1270
<210> 10
   <211> 1237
   <212> DNA
   <213> Artificial Sequence
<220>
   <223> vector sequences (partial)
<400> 10
   ttgacaatta atcatcggct cgtataatgt gtggaattgt gagcggataa caatttcaca      60
   caggagatat catatgaaaa agacagctat cgcgattgca gtggcactgg ctggtttcgc     120
   taccgttgcg caagcttctc gagttaatag agccccactg aaaagatcaa gaattctgca     180
   catggcactg acaggtgcct cagatccatc agcagaagct gaggcaaatg gtgaaaaacc     240
   ctttctgctg agagccctgc agattgccct ggtggtaagc ctgtattggg tgaccagcat     300
   ctctatggta ttcctgaaca aatatctgct ggattcccct agtctgaggc tggatactcc     360
   aatttttgtt accttttatc agtgcctggt tactaccctg ctgtgcaagg gactgtctgc     420
   tctggcagct tgctgtccag gtgcagttga ttttcctagc ctgaggctgg atctgagagt     480
   agcaagatca gtactgccac tgagtgtagt ctttattggt atgattacct tcaacaacct     540
   gtgtctgaag tatgtggggg tggcatttta caatgttggc agatcactga ccacagtttt     600
   taatgtgctg ctgagctatc tgctgctgaa acagaccaca agtttttatg ccctgctgac     660
   ctgtgggatc atcattggtg gattctggct gggagttgat caggaagggg cagaaggaac     720
   cctgagctgg ctgggcactg tttttggtgt tctggcctct ctgtgtgtta gcctgaatgc     780
   aatttacacc accaaagttc tgcctgcagt tgatggcagt atttggagac tgacttttta     840
   taataatgtt aatgcctgta tcctgtttct gccactgctg ctgctgctgg gggaactgca     900
   ggccctgagg gattttgcac agctgggttc agctcacttc tggggtatga tgaccctggg     960
   gggtctgttt ggttttgcca ttgggtatgt cacagggctg cagattaaat tcacctcacc    1020
   actgacacat aatgttagtg gcacagccaa agcctgtgcc cagactgtgc tggctgtgct    1080
   gtattatgaa gaaacaaagt cctttctgtg gtggacctca aatatgatgg tactgggtgg    1140
   cagcagtgcc tacacctggg tgagaggctg ggaaatgaaa aagaccccag aagaacccag    1200
   cccaaaagat tctgaaaaaa gtgcaatggg ggtttaa                             1237
<210> 11
   <211> 1313
   <212> DNA
   <213> Artificial Sequence
<220>
   <223> vector sequences (partial)
<400> 11
   ccacaattca gcaaattgtg aacatcatca cgttcatctt tccctggttg ccaatggccc      60
   attttcctgt cagtaacgag aaggtcgcga attcaggcgc tttttagact ggtcgtaatg     120
   aacaattctt aagaaggaga tatacatatg aaaaagacag ctatcgcgat tgcagtggca     180
   ctggctggtt tcgctaccgt tgcgcaagct tctcgagtta atagagcccc actgaaaaga     240
   tcaagaattc tgcacatggc actgacaggt gcctcagatc catcagcaga agctgaggca     300
   aatggtgaaa aaccctttct gctgagagcc ctgcagattg ccctggtggt aagcctgtat     360
   tgggtgacca gcatctctat ggtattcctg aacaaatatc tgctggattc ccctagtctg     420
   aggctggata ctccaatttt tgttaccttt tatcagtgcc tggttactac cctgctgtgc     480
   aagggactgt ctgctctggc agcttgctgt ccaggtgcag ttgattttcc tagcctgagg     540
   ctggatctga gagtagcaag atcagtactg ccactgagtg tagtctttat tggtatgatt     600
   accttcaaca acctgtgtct gaagtatgtg ggggtggcat tttacaatgt tggcagatca      660
   ctgaccacag tttttaatgt gctgctgagc tatctgctgc tgaaacagac cacaagtttt     720
   tatgccctgc tgacctgtgg gatcatcatt ggtggattct ggctgggagt tgatcaggaa     780
   ggggcagaag gaaccctgag ctggctgggc actgtttttg gtgttctggc ctctctgtgt     840
   gttagcctga atgcaatttt acaccaccaa agttctgcct gcagttgatg gcagtatttg     900
   gagactgact tttttataat aatgttaatg cctgtatcct gtttctgcca ctgctgctgc     960
   tgctggggga actgcaggcc ctgagggatt ttgcacagct gggttcagct cacttctggg    1020
   gtatgatgac cctggggggt ctgtttggtt ttgccattgg gtatgtcaca gggctgcaga   1080
   ttaaattcac ctcaccactg acacataatg ttagtggcac agccaaagcc tgtgcccaga    1140
   ctgtgctggc tgtgctgtat tatgaagaaa caaagtcctt tctgtggtgg acctcaaata    1200
   tgatggtact gggtggcagc agtgcctaca cctgggtgag aggctgggaa atgaaaaaga    1260
   ccccagaaga acccagccca aaagattctg aaaaaagtgc aatgggggtt taa           1313
<210> 12
   <211> 1346
   <212> DNA
   <213> Artificial Sequence
<220>
   <223> vector sequence (partial)
<400> 12
   ccacaattca gcaaattgtg aacatcatca cgttcatctt tccctggttg ccaatggccc      60
   attttcctgt cagtaacgag aaggtcgcga attcaggcgc tttttagact ggtcgtaatg     120
   aacaattctt aagaaggaga tatacatatg aaaaagacag ctatcgcgat tgcagtggca     180
   ctggctggtt tcgctaccgt tgcgcaagct tctcgagtta atagagcccc actgaaaaga     240
   tcaagaattc tgcacatggc actgacaggt gcctcagatc catcagcaga agctgaggca     300
   aatggtgaaa aaccctttct gctgagagcc ctgcagattg ccctggtggt aagcctgtat     360
   tgggtgacca gcatctctat ggtattcctg aacaaatatc tgctggattc ccctagtctg     420
   aggctggata ctccaatttt tgttaccttt tatcagtgcc tggttactac cctgctgtgc     480
   aagggactgt ctgctctggc agcttgctgt ccaggtgcag ttgattttcc tagcctgagg     540
   ctggatctga gagtagcaag atcagtactg ccactgagtg tagtctttat tggtatgatt     600
   accttcaaca acctgtgtct gaagtatgtg ggggtggcat tttacaatgt tggcagatca     660
   ctgaccacag tttttaatgt gctgctgagc tatctgctgc tgaaacagac cacaagtttt     720
   tatgccctgc tgacctgtgg gatcatcatt ggtggattct ggctgggagt tgatcaggaa     780
   ggggcagaag gaaccctgag ctggctgggc actgtttttg gtgttctggc ctctctgtgt     840
   gttagcctga atgcaatttt acaccaccaa agttctgcct gcagttgatg gcagtatttg     900
   gagactgact tttttataat aatgttaatg cctgtatcct gtttctgcca ctgctgctgc     960
   tgctggggga actgcaggcc ctgagggatt ttgcacagct gggttcagct cacttctggg    1020
   gtatgatgac cctggggggt ctgtttggtt ttgccattgg gtatgtcaca gggctgcaga    1080
   ttaaattcac ctcaccactg acacataatg ttagtggcac agccaaagcc tgtgcccaga    1140
   ctgtgctggc tgtgctgtat tatgaagaaa caaagtcctt tctgtggtgg acctcaaata    1200
   tgatggtact gggtggcagc agtgcctaca cctgggtgag aggctgggaa atgaaaaaga    1260
   ccccagaaga acccagccca aaagattctg aaaaaagtgc aatgggggtt agatctgtcg    1320
   actacaagga cgatgacgac aagtga                                          1346